# EUROPEAN PATENT APPLICATION

(11) **EP 3 653 719 A1**
(43) Date of publication of application: **20.05.2020**
(21) Application number: 18206733.0
(22) Date of filing: 16.11.2018
(51) Int. Cl.: C12P 5/02

(54) **A METHOD OF PRODUCING BIOMASS DEGRADATION PRODUCTS**

(71) Applicant: NGF Nature Energy Biogas A/S, 5220 Odense SØ (DK)
(72) Inventor: JEPPESEN, Martin Dan, 5200 Odense V (DK); GYLLENBORG, Morten Enzo, 5700 Svendborg (DK)
(74) Representative: Patentgruppen A/S

(57) **Abstract**

A method of producing biomass degradation products from soft biomass is disclosed, the method comprising the steps of
- providing a soft biomass,
- pretreating the soft biomass in a pretreatment step at a pressure below 2 bar by heating the soft biomass to at a pretreatment temperature between 65 to 100 degrees Celsius to obtain a pretreated biomass,
- hydrolyzing the pretreated biomass in a first hydrolyzation step to obtain a biomass hydrolysate, and
- posttreating the biomass hydrolysate in a pressurized posttreatment step by heating the biomass hydrolysate to a posttreatment temperature above 150 degrees Celsius to obtain a posttreated biomass,
- hydrolyzing the posttreated biomass in a second hydrolyzation step,
wherein biogas is obtained from at least the first hydrolyzation step or the second hydrolyzation step.

## Description

### FIELD OF INVENTION

The invention relates to a method of producing biomass degradation products, such as biogas, from soft biomass.

### BACKGROUND

The major constituents of soft biomass include lignin, cellulose and hemicellulose, which may be degraded to produce biogas. However, it is difficult to degrade soft biomass, and one or more severe pretreatment steps have previously been necessary in order to deconstruct biomass and facilitate e.g. microbes access to cellulose and hemicellulose. Cellulose is for example comprised of a glucose-linked structure that is resistant to degradation due to the number of hydrogen bonds in its crystalline structure.

Steam explosion is an example of pretreatment, where biomass is heated at high pressure with steam and then brought back at atmospheric pressure causing an explosive decompression that cause the disruption of the biomass fibers. Other procedures, including chemical processing with strong acids, high pressures, or high temperatures, are generally employed to degrade cellulose to glucose. However, in addition to rendering the cellulose and hemicellulose of the biomass accessible for degradation with increasing temperature and pressure, there is the drawback that also many process inhibitors, such as furfural 5-HMF and acetic acid, are formed with the increased temperature.

Thus, it is necessary to balance between requiring a high temperature to make the hemicellulose and cellulose accessible for degradation and on the other hand not raising the temperature too much, since this leads to the formation of more inhibitory compounds, which means that either a less than optimal amount of cellulose and hemicellulose is made accessible for degradation or too many process inhibitory compounds are formed, which e.g. limits the biogas yield.

Hence a simple and less time-consuming process is desired, which increases the accessibility of hemicellulose and cellulose for degradation to increase the yields of degradation products, such as biogas, while decreasing the amounts of inhibitory compounds formed during the process.

It is an object of the present invention to solve one or more of the above problems.

### SUMMARY

The invention relates to a method of producing biomass degradation products from soft biomass, the method comprising the steps of
- providing a soft biomass,
- pretreating the soft biomass in a pretreatment step at a pressure below 2 bar by heating the soft biomass to at a pretreatment temperature between 65 to 100 degrees Celsius to obtain a pretreated biomass,
- hydrolyzing the pretreated biomass in a first hydrolyzation step to obtain a biomass hydrolysate, and
- posttreating the biomass hydrolysate in a pressurized posttreatment step by heating the biomass hydrolysate to a posttreatment temperature above 150 degrees Celsius to obtain a posttreated biomass,
- hydrolyzing the posttreated biomass in a second hydrolyzation step,
   wherein biogas is obtained from at least the first hydrolyzation step or the second hydrolyzation step.

By employing a pretreatment step at a pressure below 2 bar by heating the soft biomass to at a pretreatment temperature between 65 to 100 degrees Celsius and a pressurized posttreatment step by heating the biomass hydrolysate to a posttreatment temperature above 150 degrees Celsius several advantages can be obtained, such as improved biogas yield, in the form of e.g. methane and carbon dioxide, decrease in process time and decrease in the formation of process inhibitors, such as furfural, 5-HMF and acetic acid.

Soft biomass, such as e.g. wheat straw, will tend to float to the top of the reactor create a thick layer of no digested wheat straw (floating layer) in an otherwise wet solution, which decrease the biomass degradation process and hinder the agitation. This problem may advantageously be solved by the present invention, which allows the biomass to be more homogeneous instead of forming a more solid layer floating on top of a more liquid layer. This allows for a more efficient biomass degradation and higher production of degradation products, such as biogas. The floating layers may especially be prevented by pretreating the soft biomass in a pretreatment step at a pressure below 2 bar by heating the soft biomass to at a pretreatment temperature between 65 to 100 degrees Celsius.

A further advantage of the invention is that it makes the soft biomass more accessible for subsequent hydrolyzation for example by melting pectin and other waxes away so that e.g. the hemicellulose is more accessible for hydrolyzation.

The pretreatment step may also allow in a synergistic way the temperature in the posttreatment step to be higher than in conventional methods since less inhibitory compounds, such as furfural, 5-HMF and acetic acid, will be formed. As a result, more lignin may be melted in the posttreatment step due to the higher temperature of more than 150 degrees Celsius, such as e.g. more than 180 degrees Celsius, allowing for a more efficient degradation of cellulose especially in the posttreatment step, allowing for obtaining higher yields of biomass degradation products, such as biogas.

The first hydrolyzation step is usually carried out with a volume load of more than 4 and often more than 5 - 6 kg VS / m3 / day.

Furthermore, the TS content is usually lower in the second hydrolyzation step compared to the TS content in the first hydrolyzation step.

According to an advantageous embodiment of the invention, the pretreatment step (NPS) is non-pressurized.
All of the above advantages are also obtained when the pretreatment step is non-pressurized, and in addition the method and especially the pretreatment step may be performed easier (for example in a continuous way) and cheaper, when the pretreatment step is non-pressurized, since it may for be performed in an open system or may be performed in reactors with over-/underpressure valves. When the pretreatment step is non-pressurized, the severity of the pretreatment is also lower than when a higher pressure is applied, and thus milder conditions are obtained, which may lead to e.g. even less formation of inhibitory compounds.

According to an advantageous embodiment of the invention, the method further comprises separating the biomass hydrolysate in a first separation step (SS) into a solid fraction and a liquid fraction.

It may be very advantageous to separate the biomass hydrolysate obtained from the first hydrolyzation step, into a solid fraction and a liquid fraction prior to the posttreatment step. The liquid fraction may then be discarded, or it may advantageously be reused in the process, for example by conveying it into the pretreatment step or reuse it later in the second hydrolyzation step, for example to adjust the aqueous content or to accelerate the process for example by catalyzation of the process due to bacteria, acid or fungi present in the liquid fraction. It may also be an advantage simply to discard the liquid fraction if the liquid fraction e.g. comprises any undesired compounds, process inhibitors or degradation products. The solid fraction is then usually the fraction employed in the further process steps and thus subsequent subjected to the posttreatment step.

The separation may for example be performed by pressing or decanting.

According to an advantageous embodiment of the invention, the method further comprises separating the posttreated biomass in a second separation step (SSS) into a solid fraction and a liquid fraction.

It may also be very advantageous to separate the posttreated biomass obtained from the second hydrolyzation step, into a solid fraction and a liquid fraction. The liquid fraction may then be discarded or it may advantageously be reused in the process, especially when the process is continuous, for example by conveying it into the pretreatment step or in the second hydrolyzation step, for example to adjust the aqueous content or to accelerate the process for example by catalyzing the process due to bacteria, acid or fungi present in the liquid fraction. It may also be an advantage simply to discard the liquid fraction if the liquid fraction e.g. comprises any undesired compounds, process inhibitors or degradation products.

According to an advantageous embodiment of the invention, the method further comprises recirculation of at least a part of a liquid fraction from any separation step to any hydrolyzation step and/or the pretreatment step.

It may be particularly useful to recirculate a part of or the whole liquid fraction separated in the first or the second separation step or both. The separated liquid fraction may in this way serve as a diluter to adjust the content of liquid in the soft biomass to a desired level or it may also serve as an accelerator to accelerate the process steps by for example catalyzing one or more hydrolysis steps. For example, the liquid fraction from the first separation step may be recirculated into the first hydrolyzation step, or the liquid fraction from the first separation step may be recirculated into the second hydrolyzation step, or the liquid fraction from the second separation step may be recirculated into the second hydrolyzation step, or the liquid fraction from the second separation step may be recirculated into the pretreatment step. It may also be especially advantageous to employ a part or all of the liquid fraction separated in any separation step in other process steps, such as in a hydrolyzation step or pretreatment step, when a continuous process according to an embodiment of the invention is performed.

According to an advantageous embodiment of the invention, biogas is obtained from both the first hydrolyzation step and from the second hydrolyzation step.

Biogas may be obtained from one or several of the process steps, however the largest amounts may be obtained when biogas is obtained from both the first and second hydrolyzation step. A pretreatment step involving heating the soft biomass to at a pretreatment temperature between 65 to 100 degrees Celsius in a non-pressurized pretreatment step and posttreating the biomass hydrolysate in a pressurized posttreatment step by heating the biomass hydrolysate to a posttreatment temperature above 150 degrees Celsius may give rise to an exceptionally high yield of biogas. This may be due to a higher liberation of hemicellulose and cellulose accessible for degradation, without the drawback that also many process inhibitors are formed during the process conditions.

According to an advantageous embodiment of the invention, at least 65% hemicellulose is degraded.

In an embodiment of the invention, at least 80% hemicellulose is degraded, such as at least 90%, or between 70 to 99%, such as between 80 to 99%. The major constituents of soft biomass are lignin, hemicellulose and cellulose. In embodiments according to the invention an unusual high amount of hemicellulose may be liberated for degradation and subsequent degraded. The hemicellulose may be degraded in various ways, for example by hydrolysis, which may be catalyzed for example by acid, bacteria or fungi. The hemicellulose may for example be degraded into sugar monomers such as xylose and arabinose and/or it may advantageously be degraded into methane.

According to an advantageous embodiment of the invention, at least 60% cellulose is degraded.

In an embodiment of the invention at least 70% cellulose is degraded, such as at least 80%, such at least 90%, or at least 95% cellulose is degraded. Alternatively, between 60% to 99% or 70% to 99% cellulose is degraded.

As with the hemicellulose, the cellulose is not readily accessible for degradation, for example due to lignin and waxes that are surrounding and binding the cellulose and/or hemicellulose. However, the process according to embodiments of the invention gives rise to a high yield of cellulose degradation.

The cellulose may for example be degraded into sugar monomers such as glucose and/or it may advantageously be degraded into methane.

According to an advantageous embodiment of the invention, the amount of hemicellulose in the biomass hydrolysate is less than 40% by weight of the amount of hemicellulose in the soft biomass.

In other words, after the first hydrolyzation step 60% or more of the hemicellulose, which was comprised in the soft biomass has been degraded.

According to an advantageous embodiment of the invention, the amount of cellulose in the biomass after the second hydrolyzation step is less than 40% by weight of the amount of cellulose in the posttreated biomass.

According to an embodiment of the invention, the amount of hemicellulose in the biomass hydrolysate relative to the amount of hemicellulose in the soft biomass is reduced more than the amount of cellulose in the biomass hydrolysate relative to the amount of cellulose in the soft biomass.

Thus, percentwise more hemicellulose than cellulose is degraded in the pretreatment and first hydrolyzation step.

According to an advantageous embodiment of the invention, furfural and 5-HMF and 2-furioc acid are generated in a combined amount of less than 5% by weight.

In an embodiment of the invention, process inhibitors, such as furfural and 5-HMF and 2-furioc acid is generated in a combined amount of less than 2.5% or less than 1%. Less than 1% process inhibitors may be formed in the pretreatment step. Alternatively, they are formed in an amount of between 0.1 to 5% or 0.1 to 2.5% The percentages are referring to by weight of total dry matter weight i.e. w/w, and e.g. 10 g inhibitor/kg dry matter is thus equal to 1%.

Process inhibitors such as furfural, 5-HMF (hydroxy methyl furfural) and 2-furioc acid may be formed as degradation products from hemicellulose and cellulose present in soft biomass. They are often unwanted side products in biomass degradation, especially when other degradation products such as biogas are desired, since they may inhibit the degrading action of for example fermentative bacteria or fungi. In contrast to conventional processes, the process according to the present invention may lead to a significant lower formation of process inhibitors such as furfural and 5-HMF and 2-furioc acid.

According to an advantageous embodiment of the invention, xylose is obtained from the first hydrolyzation step.

According to an advantageous embodiment of the invention, lignin is obtained from the second hydrolyzation step in a purity of more than 30%.

In embodiments of the invention, lignin is obtained from the second hydrolyzation step in a purity of more than 40%, such as in a purity of more than 50% purity. Alternatively, lignin is obtained from the second hydrolyzation step in a purity of between 30 to 50% purity. The purity is calculated VS.

According to an embodiment of the invention, glucose is obtained from the second hydrolyzation step, when for example glucose producing enzymes have been employed in the second hydrolyzation step.

According to an advantageous embodiment of the invention, the pretreatment temperature is between 65 to 90 degrees Celsius, such as between 65 to 80 degrees Celsius or between 70 to 90 degrees Celsius. The advantage of a pretreatment temperature above 65 degrees Celsius is that e.g. waxes and pectins can melt so it makes the biomass more accessible to subsequent hydrolysis/digestion. From an economical viewpoint, the temperature in the pretreatment step may preferable lie around 70 degrees Celsius. Higher temperatures may also be efficient; however it requires more energy to obtain. Temperatures higher than 100 degrees Celsius are not desired, so as not to reach too harsh conditions, which can lead to formation of process inhibitors such as e.g. furfural and 5-HMF.

According to an advantageous embodiment of the invention, the posttreatment temperature is between 150 to 230 degrees Celsius, such as between 170 to 210 degrees Celsius, such as between 180 to 200 degrees Celsius.

A significant advantage of the invention may be that not so many process inhibitors are formed, which means that the temperatures of the posttreatment step may be higher, such as higher than 150 degrees Celsius or higher than 170 degrees Celsius or even higher than 180 degrees Celsius, and still leading to a very high conversion of biomass as more lignin may be melted away under the high process temperatures meaning that more cellulose may be hydrolyzed, and thus more biogas produced.

A further advantage of the invention may be that the degradation of biomass may be obtained in a short amount of time, while still obtaining very high yield of various desired degradation products, such as biogas.

According to an advantageous embodiment of the invention, the pretreatment step is performed for 2 hours or less.

In an embodiment of the invention, the pretreatment step is performed for 1 hour or less, such as 45 minutes or less, such as 30 minutes or less, or 15 minutes or less. Alternatively, the pretreatment has a duration of between 5 minutes to 2 hours, such as between 5 minutes and 1 hour.

An advantage of the invention may thus be that the pretreatment step may be very effective in conditioning the biomass to make it more homogeneous and more accessible for further degradation, even when performed for only a short time, which may make the process very cost-efficient.

The pretreatment step may usually be performed as a batch process, but may also be performed as a continuous process, which may be very convenient.

According to an advantageous embodiment of the invention, the first hydrolyzation step is performed for less than 20 days, such as less than 10 days.

The first hydrolysis step may only require to be performed for a short time, such as less than 10 days or even less or such as between 1 to 10 days. When the process for example is performed as a continuous process, where biomass is continuously conveyed into the first hydrolyzation step, and biomass is continuously removed from the first hydrolyzation step, or in the case where only a fraction at a time is removed from the first hydrolyzation step, the time is referring to an average retention time in the hydrolyzation step.

When the retention time is below approximately 14 days, the bacteria usually do not have time for enough cell division and it may thus not be possible to ensure a high enough bacteria concentration, which is also known as washing out the bacteria. To ensure a stable and high enough concentration of bacteria, some of the liquid after the separation can advantageously be recirculated back to the first hydrolyzation step.

A short retention time below 10 days may also lead to a high organic loading measured as: new organic material / volume / day. Normally the organic loading needs to be under 7 kg organic material / M3 reactor volume / 24 hours, and liquid from a separation step may thus advantageously be recirculated into the process, e.g. first hydrolyzation step, again
According to an advantageous embodiment of the invention, the posttreatment step is performed for less than 1 hour.

In an embodiment of the invention, the posttreatment step is performed for less than 45 minutes, such as less than 30 minutes, or alternatively between 10 to 30 minutes. The advantages of the posttreatment step may be very efficiently obtained in a short amount of time, which especially in combination with the pretreatment step, which may also only require to be performed for a short time interval, leads to a very efficient process, which may reduce the costs.

According to an advantageous embodiment of the invention, the second hydrolyzation step is performed for less than 30 days.

In an embodiment of the invention, the second hydrolyzation step is performed for less than 25 days, such as less than 20 days, such as less than 15 or less than 10 days. Alternatively, between 1 to 30 days. The time is here referring to the average retenton time in the second hydrolyzation step.

According to an advantageous embodiment of the invention, the first hydrolyzation step is performed by bacteria, enzymes and/or fungi.

The hydrolyzation taking place in the first hydrolyzation step may be catalyzed in various ways. For example, by bacteria, fungi or enzymes, which may be added or may already be present in the biomass or possibly comprised in food waste, which may advantageously be added to the biomass. Depending on which way the hydrolysis is catalyzed, different degradation products may be obtained. For example, bacteria may give methane or lactic acid or other acids. Enzymatic hydrolysis may give sugar oligomers or sugar monomers, such as glucose, xylose and arabinose. Fungi may also give sugars or alcohols.

According to an advantageous embodiment of the invention, the second hydrolysis step is performed by bacteria, enzymes and/or fungi.

The hydrolyzation taking place in the second hydrolyzation step may also be catalyzed in various ways. For example, by bacteria, fungi or enzymes, which may be addedor may already be present in the biomass or possibly comprised in food waste, which may advantageously be added to the biomass. Depending on which way the hydrolysis is catalyzed, different degradation products may be obtained.

According to an advantageous embodiment of the invention, the enzymes are hydrolyzing enzymes, such as xylanases, cellulases, pectinases, lipases, arabinases or any combination thereof.

According to an advantageous embodiment of the invention, the bacteria are fermentative bacteria, such as ruminococcus albus, ruminococcus flavefaciens and/or fibrobacter succinogenes.

According to an advantageous embodiment of the invention, acid and/or food waste is added to the soft biomass.

Food waste added or comprised in the biomass may both promote a more homogeneous biomass, especially in the pretreatment step and it may also catalyze hydrolysis of the biomass. Without being bound to any specific theory it may be due to an acidic pH of the food waste, which may be there from the beginning or develop as the food waste decomposes. The food waste may thus advantageously be acidic, such as rotten or fermented food waste. In this way the food waste may naturally develop lactic acid, which may be used to acidify the soft biomass. Alternatively, acid may also be added to the biomass. The acid may be any kind of acid, such as strong or weak acid, organic or inorganic acid. The acid may for example be a mineral acid such as hydrochloric acid or sulfuric acid.

According to an advantageous embodiment of the invention, the acid is a weak acid.

If the acid is a weak acid, such as oxalic acid, it may provide some special benefits, such as providing high yields of biogas and short process times, in particular in the pretreatment step and the first hydrolysis step, and it may make the biomass more homogeneous and more accessible to degradation. The weak acid may for example be an organic acid, such as lactic acid or acetic acid. The pKa of the acid may advantageously be between 1 to 7, such as 1 to 6 or 1 to 5.

According to an advantageous embodiment of the invention, the soft biomass is straw, corn stover, bagasse or any combination thereof. Straw may for example be wheat straw or seed grass straw. These examples of biomass may be especially suitable and give high yields of desired degradation products, such as e.g. biogas.

According to an advantageous embodiment of the invention, the pH in the pretreatment step is between 2 to 9. In a further embodiment of the invention, the pH is between 3 to 7, such as between 3 to 4.

In an embodiment of the invention, the pH in the first and/or second hydrolysis step is between 2 to 10. The pH may be dependent on whether the biomass is hydrolyzed with bacteria, where a pH of 7 to 10 may be suitable or with enzymes, where a pH of 4 to 6 may be suitable, or acid, where a pH of 2 to 4 may be advantageous.

In an embodiment of the invention, the pH in the posttreatment step is between 2 to 10, which may depend on whether it prior to the posttreatment step has been hydrolyzed with bacteria, in which case the pH may be 7 to 10, or enzymes, in which case the pH may be 4 to 6 or acid, in which case the pH may be 2 to 4.

According to an advantageous embodiment of the invention, the pressure is between 5 to 25 bar in the pressurized posttreatment step.

In an embodiment of the invention, the pressure is between 8 to 20 bar in the posttreatment step, such as between 10 to 15 bar. The pressure may advantageously be obtained by thermal treatment. In conventional methods of biogas degradation, mechanical pressure is normally necessary, but in embodiments according to the invention this is not necessary, which may be an advantage since pressure applied by mechanical means, such as by pelletising or briqueting is often an expensive procedure.

According to an embodiment of the invention, the method is a continuous process. All the process steps may be performed in a continuous way, especially the pretreatment, first hydrolyzation step and second hydrolyzation step may in particular be suitable for this, which may be convenient and cost-effective. A liquid part of the hydrolyzed biomass possibly obtained from a first separation step may for example be used in the second hydrolysis step to dilute the output from the posttreatment.

According to an advantageous embodiment of the invention, the methane yield is more than 280 M3 per ton of soft biomass VS.

In an embodiment of the invention, the biogas yield is more than 310 M3 per ton of soft biomass VS, such as more than 340, or such as more than 370 M3 per ton of soft biomass VS entering the process.

According to an embodiment of the invention, a system is arranged to operate according to the method of the invention or any of its embodiments.

### Definitions

As used herein, the singular forms "a", "an" and "the" include plural referents unless the context clearly dictates otherwise.

As used herein, "at least one" is intended to mean one or more, i.e. 1, 2, 3, 4, 5, 6, 7, 8, 9, 10, etc.

As used herein, the term "biomass" is intended to mean material of organic origin.

As used herein, the term "biomass degradation products" shall mean any product stemming from degradation of biomass, such as C5- and C6-sugar monomers and oligomers, biogas, furfural, and 5-HMF.

As used herein, the term "biogas" is intended to mean methane gas and carbondioxide gas obtained from degradation of biological material, such as biomass.

As used herein, the term "non-pressurized" is intended to mean ambient pressure, which at average sea level is a pressure around 1 bar = 101 kPa.

As used herein, the term "pressurized" is intended to mean a pressure significantly higher than ambient pressure. Usually this may be around 10-20 bar, especially in the field of biogas production.

As used herein, the term "inoculum" shall mean material containing bacteria, such as degassed biomass from an existing biogas facility or animal waste.

As used herein, the term "soft biomass" is intended to mean cellulosic and herbaceous types of biomass, such as wheat straw, corn stover, rice straw, grass, and bagasse.

As used herein, the term "dry matter" is intended to mean the residual when water is evaporated.

As used herein, the term volatile solids (VS) shall mean the organic part of dry matter. Usually this is measured by heating a sample (which has been dried at 105 degrees Celsius) to 450 degrees Celsius, so that only salts and ashes remain.

As used herein the "first separation step" and "second separation step" are terms intended to refer to two different separation steps of particular interest herein. It is noted that "first" and "second" are only intended as labels for convenient reference to such particular separation steps, and is without any special meaning other than such labelling. Herein, more separation steps may be given without any such labelling, but could thus be considered as e.g. third, fourth, or fifth etc. weight-ratios if so intended, without any special meaning other that convenient labelling.

As used herein, the term "liquid fraction" is intended to mean the fraction having the lowest dry matter after a separation step. The amount of suspended solids is normally around 4%, but typically varies from 0-10%.

As used herein, the term "solid fraction" intended to mean the fraction having the highest dry matter after a separation step. The amount of suspended solids is normally around 20-25%, but may vary from 10-95%.

As used herein, "hydrolyzation step" is intended to mean a step, wherein hydrolyzation occurs, however other processes may occur also in the same step, such as for example other types of degradation or cross-linking.

As used herein, "pretreatment step" is intended to mean a treatment step prior to the first hydrolyzation step. The pretreatment step is thus also always carried out before the posttreatment step.

As used herein, "posttreatment step" is intended to mean a treatment step subsequent to the first hydrolyzation step. The posttreatment step is thus also always carried out after the pretreatment step.

As used herein "Nm3" is intended to mean one (1) cubic metre of gas at 0 degrees Celsius and 1 atmosphere.

### Abbreviations

VS = volatile solids
5-HMF = 5-(hydroxymethyl)furfural
OTS = organic total solids
TS = total solids
Nm3 = normal cubic metres

### THE FIGURES

The invention will now be described with reference to the figures where
Figure 1 illustrates a general method of producing biomass degradation products, such as biogas, from soft biomass according to an embodiment of the invention.
Figure 2 illustrates a further method of producing biomass degradation products, such as biogas, from soft biomass according to an embodiment of the invention.
Figure 3 illustrates an even further method of producing biomass degradation products, such as biogas, from soft biomass according to an embodiment of the invention.
Figure 4 illustrates methane yield for raw wheat straw and heated wheat straw as a function of time according to an embodiment of the invention.
Figure 5 illustrates the effect on hemicellulose concentration and inhibitor formation as result of time of heating to 70 degrees Celsius according to an embodiment of the invention.
Figure 6 illustrates the effect on hemicellulose conversion as result of time of digestion according to an embodiment of the invention.
Figure 7 illustrates the effect of temperature and pH in the pretreatment step according to an embodiment of the invention. The first number is temperature (°C) and second is pH in the pretreatment.
Figure 8 illustrates the effect of both pretreatment and posttreatment on the methane yield according to an embodiment of the invention.

### DETAILED DESCRIPTION

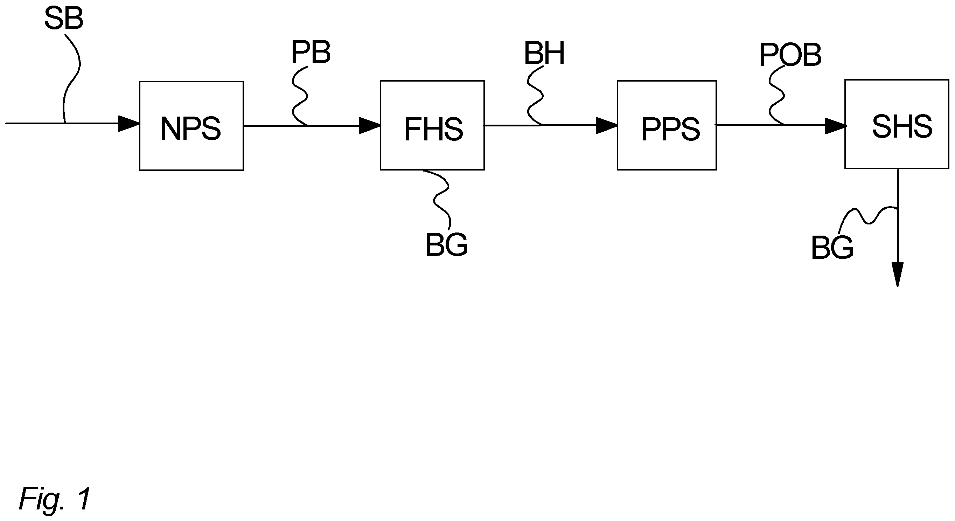

Referring to figure 1, a schematic view of a process according to an embodiment of the invention is shown.

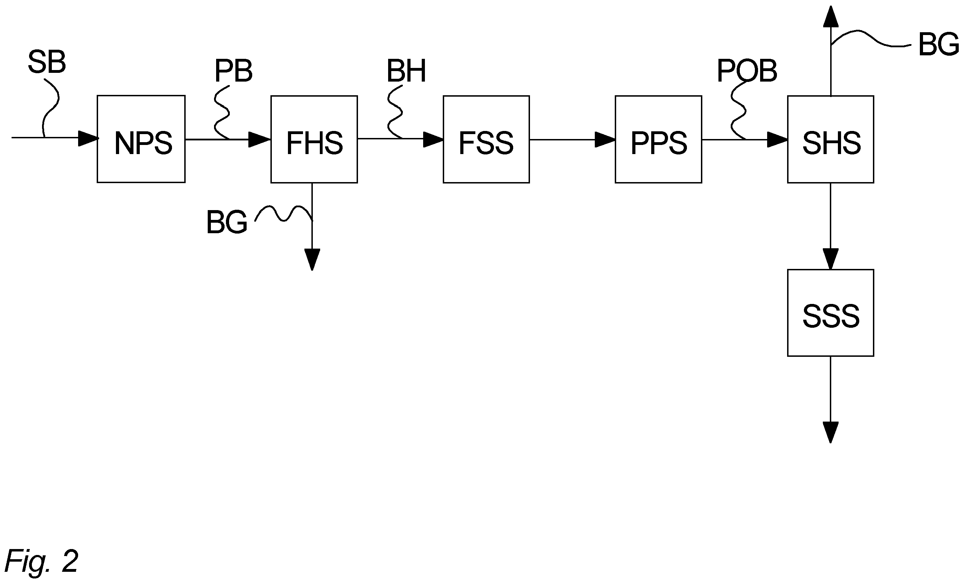

Further embodiments are illustrated in figures 2-3, and all of these embodiments may be understood in the light of figure 1 and the discussion thereof below.

Returning to figure 1, a soft biomass, which may advantageously be acidic, is provided and subjected to heating in a non-pressurized pretreatment step at a pretreatment temperature PT between 65 to 100 degrees Celsius at ambient pressure. The heating may for example be performed in a tank or container with or without stirring. The retention time in the pretreatment step is usually short, such as less than one hour or less than 30 minutes, but it may also be longer if this may be convenient. The pretreated biomass (PB) may then be conveyed into a further tank to be hydrolyzed in a first hydrolyzation step or it may in principle also be hydrolyzed in the same container as it was pretreated. The hydrolyzation may be performed without any further additives, where any acid already present may catalyze the hydrolyzation, or it may be performed for example by the addition of bacteria or fungi. The first hydrolyzation is usually performed at a pH around neutral, such as for example between pH 6-8. It may also be lower for example in the case of an acid catalyzed hydrolyzation. In the first hydrolyzation step both hemicellulose and cellulose are hydrolyzed, however relatively more hemicellulose than cellulose is hydrolyzed. The hydrolyzation products may for example be C5 sugars from hemicellulose and C6 sugars from cellulose, which may be further degraded. An important degradation product, which may be obtained from the first hydrolyzation step is biogas, comprising methane and/or carbon dioxide.

The resulting biomass hydrolysate (BH) is then posttreated in a pressurized posttreatment step, where the temperature is above 150 degrees Celsius and the pressure is above ambient, such as for example between 5 to 25 bar. The posttreatment may be performed in a closed container or tank. Subsequent to the posttreatment step, the posttreated biomass may then be subjected to a second hydrolyzation step. Biogas may advantageously be obtained from the first and/or second hydrolyzation step. The biogas from the second hydrolyzation step may be further purified and may be combined with the biogas produced in the first hydrolyzation step.

Referring to figure 2, an embodiment of the invention is shown, where the process of figure 2 includes, further to the steps of the embodiment of figure 1, a separation step after the first hydrolyzation step. The separation step, may be called a first separation step. The separation step separates the biomass hydrolysate into a solid fraction and a liquid fraction. The solid fraction is then subjected to a pressurized posttreatment step and the resulting posttreated biomass is subjected to a second hydrolyzation and thereafter it may be subjected to a second separation step leading to a liquid fraction and a solid fraction.

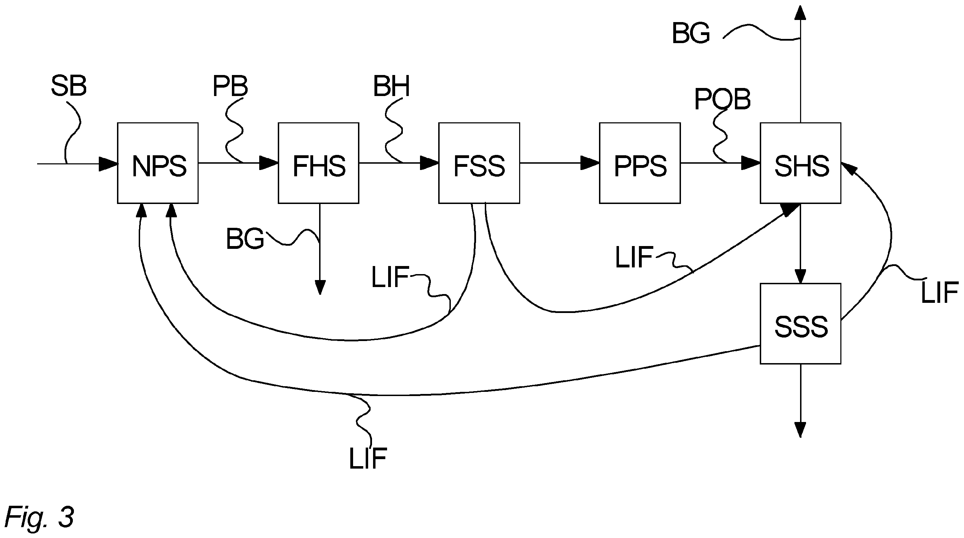

Referring to figure 3, an embodiment of the invention is shown, where the process of figure 3 includes, further to the steps of the embodiment of figure 1 or 2, that any liquid fraction LIF from any of the separation steps may be recycled into the non-pressurized pretreatment step and/or the second hydrolyzation step.

### EXAMPLES

### Example 1: The effect of pretreating a biomass before a biogas process in a hydrolyzation step.

This example describes the effects of pretreating a biomass by heating the biomass prior a biogas process in a hydrolyzation step. A fixed amount of raw wheat straw has been heated for one hour together with food waste prior the biogas process.

### Materials and methods:

### Biomass mix:

- 3.51 g of wheat straw with a dry matter of 86%
- 23.12 g of food waste with a dry matter of 13.8%
- 57.41 g of water (no dry matter)

The solution has been stirred for 5 min. Upon stirring, the solution with at pH of 4.5 was heated to 70 °C for one hour and then cooled down to 50 °C. 315.9 g of inoculum was added to the solution and the biogas process was started using the APTMS-II system from BiogasSystems.

The biogas process was conducted in 30 days at a pH of 8.5 and a temperature of 50 °C.

As controls, three samples with pure inoculum were digested as well as two samples with food waste and inoculum and one sample with raw wheat straw, i.e. six control digestions in total, see *Table 1.*

**Table 1: Samples for the first example.**

| **Sample** | **Contents** | **Mass (g)** |
|---|---|---|
| 3 x Blank | Inoculum | 400 |
| | | |
| 2 x Food waste control | Inoculum | 400 |
| | Food waste | 58.5 |
| | | |
| 1 x Wheat straw control | Inoculum | 284.7 |
| | Wheat straw | 6.3 |
| | Water | 108.9 |
| | | |
| 1 x Heat treated wheat straw and food waste | Inoculum | 316.0 |
| | Food waste | 23.1 |
| | Wheat straw | 3.5 |
| | Water | 57.4 |

### Results

Soft biomass, in this case wheat straw, will, if not pretreated, tend to create floating layers in an otherwise wet solution, inhibiting the biogas process. Heating the wheat straw together with food waste allows the biomass to be diluted in the solution, instead of floating on top of the biomass mix, allowing a more efficient anaerobic digestion process afterwards.

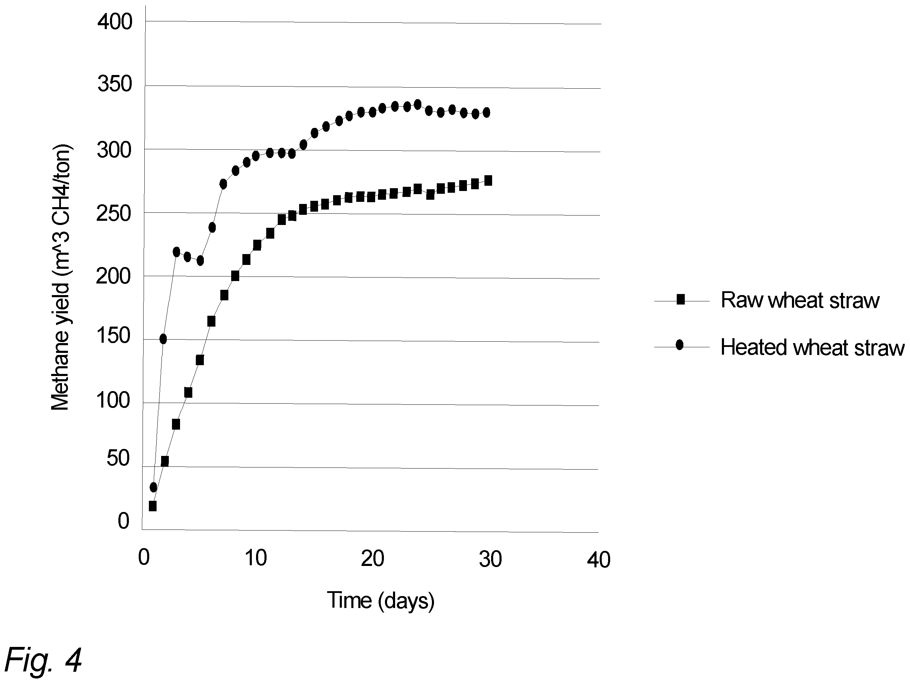

To quantify the effect of heating the wheat straw together with the food waste, total methane production was measured from the mix, upon which, the contribution to the methane production from the inoculum and the food waste were subtracted, taking into account the results from controls. A large increase of 20% on the methane yield was observed compared to the raw wheat straw sample, see Figure 4. The corresponding numbers of Figure 4 are listed in Table 2.

**Table 2. Methane yield versus time**

| | **Methane yield (m³ CH₄/ton)** | |
|---|---|---|
| **Time (days)** | **Raw wheat straw** | **Heated wheat straw** |
| 0 | 0 | 0 |
| 1 | 10 | 94 |
| 2 | 42 | 148 |
| 3 | 66 | 217 |
| 4 | 89 | 214 |
| 5 | 111 | 211 |
| 6 | 140 | 237 |
| 7 | 159 | 271 |
| 8 | 172 | 281 |
| 9 | 184 | 288 |
| 10 | 193 | 294 |
| 11 | 201 | 295 |
| 12 | 210 | 294 |
| 13 | 214 | 295 |
| 14 | 216 | 303 |
| 15 | 218 | 311 |
| 16 | 219 | 317 |
| 17 | 220 | 322 |
| 18 | 220 | 325 |
| 19 | 220 | 328 |
| 20 | 220 | 328 |
| 21 | 221 | 331 |
| 22 | 222 | 332 |
| 23 | 223 | 333 |
| 24 | 225 | 335 |
| 25 | 219 | 329 |
| 26 | 221 | 329 |
| 27 | 223 | 330 |
| 28 | 224 | 329 |
| 29 | 224 | 328 |
| 30 | 226 | 329 |

### Example 2: Hemicellulose conversion

Heating wheat straw in a pretreatment step damages the structure of the biomass and makes the hemicellulose more accessible without producing a large concentration of inhibitors such as furfural and 5-HMF which a pretreatment at higher temperatures does. This example describes the effect on hemicellulose conversion during the pretreatment step and the first hydrolyzation step.

### Materials and methods:

Four samples are prepared with wheat straw, citric acid and water and stirred for 5 min. The solutions are heated for 0 min, 15 min, 30 min, 45 min or 1 hour, respectively, at 70 °C, and the hemicellulose concentration and the inhibitor concentration are measured at the different time points.

Five new samples are prepared with wheat straw, citric acid and water and stirred for 5 min, heated for 1 hour at 70 °C and cooled down to 50 °C. The solutions are adjusted to a pH of 8 with NaOH and afterwards mixed with inoculum and a first hydrolyzation step comprising an anaerobic digestion process is started. The first hydrolyzation, here a biogas process, is stopped after 12 days, and the hemicellulose concentrations and the inhibitor concentrations are measured in each of the samples.

The hemicellulose and inhibitor concentrations are also measured in samples with pure inoculum and with raw wheat straw and inoculum.

### Results

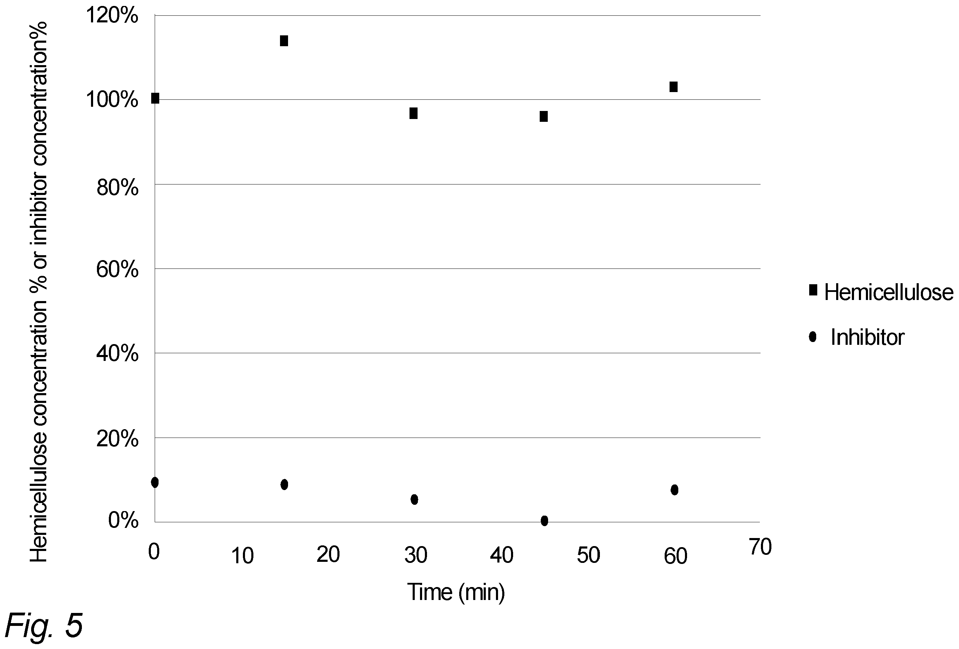

Hemicellulose was not decomposed during the heat/acid treatment as illustrated in figure 5. The corresponding numbers of Figure 5 are listed in Table 3.

**Table 3.**

| **Minutes** | **Hemicellulose** | **Inhibitor** |
|---|---|---|
| 0 | 100% | 9% |
| 15 | 114% | 9% |
| 30 | 96% | 5% |
| 45 | 96% | 0% |
| 60 | 103% | 7% |

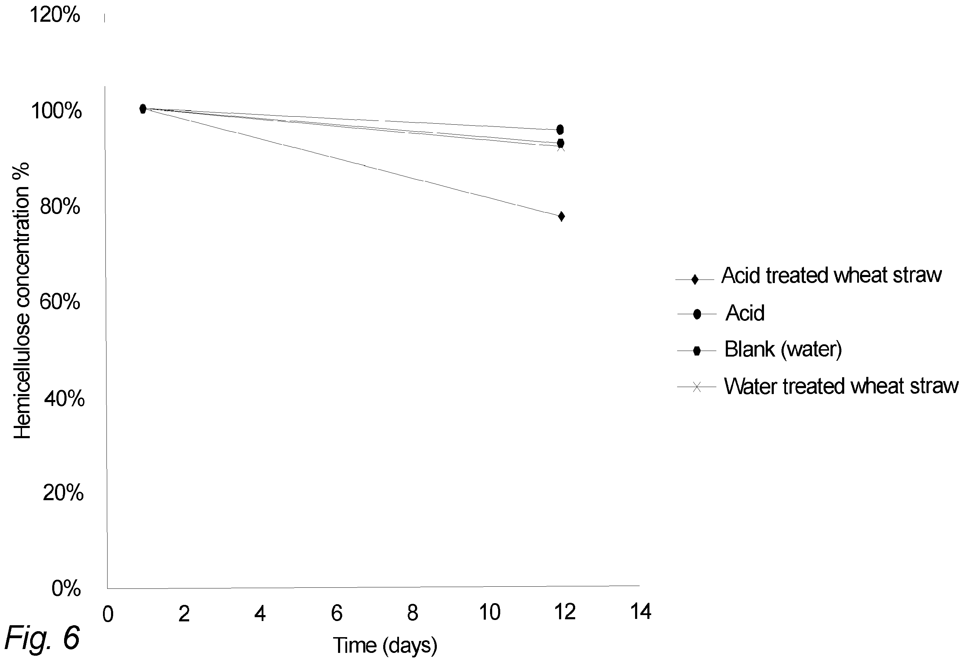

The acid/heat treatment did open the wheat straw structure. 23% of the hemicellulose is converted for the heat/acid treated sample where only 8% of the hemicellulose was converted for the not treated sample, see figure 6. The corresponding numbers of Figure 6 are listed in Table 4. The inhibitor concentration was below the detection limit (0.1 g/kg).

**Table 4.**

| | **Hemicellulose concentration** | | | |
|---|---|---|---|---|
| **Time (days)** | **Acid treated wheat straw** | **Acid** | **Blank (water)** | **Water treated wheat straw** |
| 1 | 100.0% | 100% | 100% | 100% |
| 12 | 76.8% | 95% | 92% | 92% |

### Example 3: Effect of pretreatment severity in the pretreatment step on the biogas yield

This example describes the effect of pretreatment severity on the biogas yield (here methane). The pretreatment severity is described by temperature, pH and time. This example evaluates the effect of pH and temperature. The temperature is varied from 80 °C to 100 °C and the pH from 2 to 9.5. The experiment is done using a statistic experimental design.

### Materials and methods

12 samples are prepared with wheat straw, Phosphorbuffer and stirred for 5 min. The solutions are heated for 1 hour. In total, eight combinations of temperature and pH are tested, in which two combinations are triplicated, see *Table* . All samples are cooled down after heat treatment, adjusted to a pH of 8 and then mixed with inoculum. Upon this, a biogas process is conducted for 12 days for each sample. The methane yields are measured.

**Table 5: Experimental setup with different combinations of pH and temperature.**

| **Sample no.** | **Temperature (°C)** | **pH** |
|---|---|---|
| 1 | 100 | 2 |
| 2,3,4, | 80 | 4.5 |
| 5 | 100 | 7 |
| 6,7,8 | 80 | 9.5 |

### Results

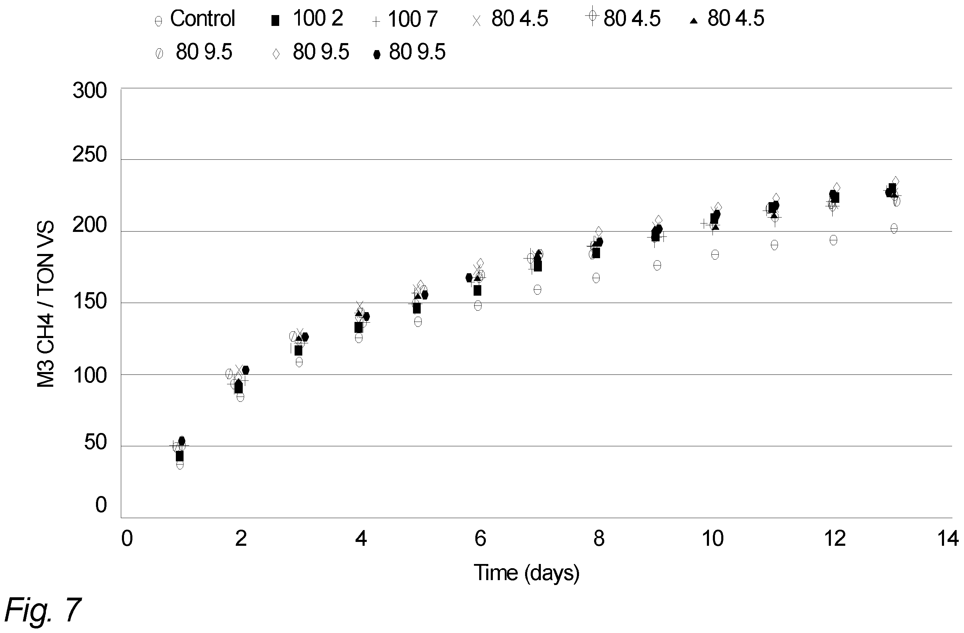

All the treated samples showed increase in biogas yield compared to the control, see figure 7. The corresponding numbers of Figure 7 are listed in Table 6 below.

**Table 6.**

| | | | **m³ CH₄/ton VS** | |
|---|---|---|---|---|
| **Sample** | **Temp (°C)** | **pH** | **Day 1** | **Day 13** |
| 1 | 100 | 2 | 47 | 231 |
| 2 | 80 | 4.5 | 48 | 231 |
| 3 | 80 | 4.5 | 46 | 224 |
| 4 | 80 | 4.5 | 49 | 224 |
| 5 | 100 | 7 | 45 | 226 |
| 6 | 80 | 9.5 | 48 | 235 |
| 7 | 80 | 9.5 | 47 | 230 |
| 8 | 80 | 9.5 | 47 | 230 |
| Control | | | 38 | 202 |

### Example 4: The effect of pretreatment severity in the second hydrolyzation step on the biogas yield

This example describes the effect of pretreatment severity on the second hydrolyzation step on the biogas, in this case methane, yield. The pretreatment severity is described by temperature, pH and time. This example evaluates the effect of pH and temperature. The temperature is varied from 130 °C to 210 °C and the pH from 2 to 12. The experiment was conducted using a statistic experimental design.

### Materials and methods

12 samples were prepared with wheat straw, citric buffer pH 4.5 and stirred for 5 min. The solutions were heated for 1 hour to 70 °C, then cooled to 50 °C, upon which inoculum were added. A second hydrolyzation step, comprising a biogas process, was conducted for 14 days for each of the samples. The samples were separated into a liquid fraction and a fiber fraction. The liquid fraction was transferred back to the biogas reactors. The fiber fractions were adjusted with phosphor buffer to reach the pH and heated for 15 min at 140, 170 or 200 °C, see Table.

**Table 7: Experimental setup with different combinations of pH and temperature.**

| **Flask no.** | **Temperature (°C)** | **pH** |
|---|---|---|
| 1 | 140 | 12 |
| 2 | 170 | 12 |
| 3 | 200 | 12 |
| 4 | 140 | 7.5 |
| 5 | 170 | 7.5 |
| 6 | 200 | 7.5 |
| 7 | 140 | 3 |
| 8 | 170 | 3 |
| 9 | 200 | 3 |
| 12 | Control, no treatment | |

Afterwards, the samples are cooled down, the pH was adjusted to pH 8 and mixed with inoculum and a biogas process in a second hydrolyzation step is conducted for 20 days for each sample. As it did take 3 days to heat all the solid fiber fraction samples the biogas process was conducted for 3 days without fibers.

The methane yields are measured after the biogas process.

### Results

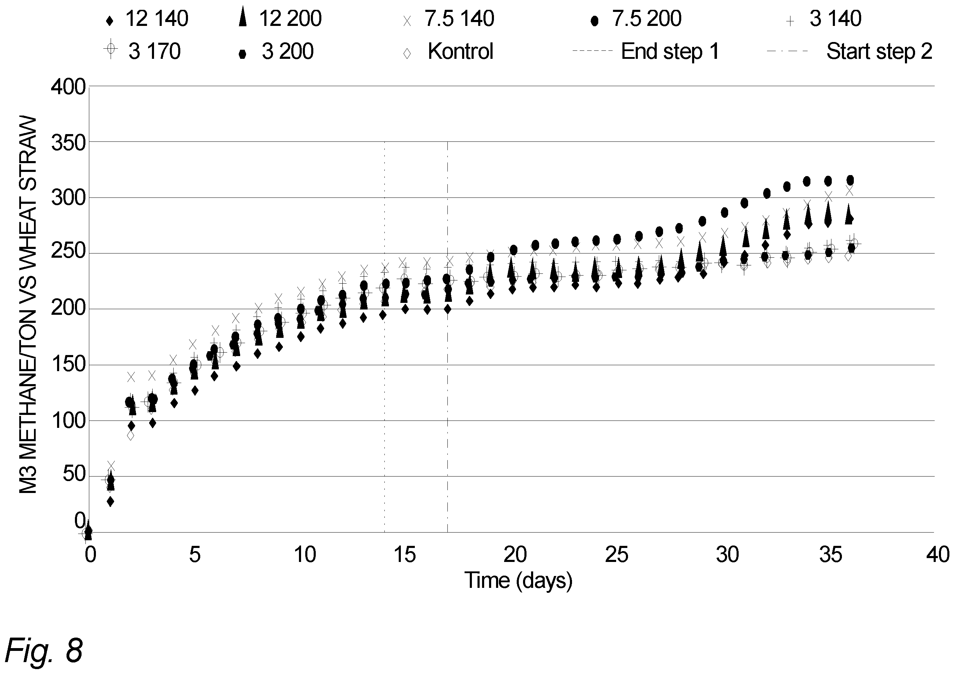

The result can be seen in figure 8. Both temperature and pH have an effect. Surprisingly the data show that the treatment at neutral pH gave the highest biogas production, the reason for that may possible be due to formation of inhibitors at low and high pH, since the process is performed batch-wise and not continuous. The corresponding numbers of Figure 8 are listed in Table 8 below.

**Table 8.**

| | | | **m³ CH₄/ton VS** | |
|---|---|---|---|---|
| **Sample** | **pH** | **Temp (°C)** | **Day 1** | **Day 36** |
| 12 140 | 12 | 140 | 28 | 281 |
| 12 200 | 12 | 200 | 48 | 289 |
| 7.5 140 | 7.5 | 140 | 59 | 309 |
| 7.5 200 | 7.5 | 200 | 46 | 317 |
| 3 140 | 3 | 140 | 48 | 262 |
| 3 170 | 3 | 170 | 47 | 262 |
| 3 200 | 3 | 200 | 50 | 257 |
| Control | | | 39 | 248 |

### Example of Biogas production

Wheat straw, food waste and water is conveyed into a pretreatment tank, where it is heated at a temperature of around 70 degrees Celsius at ambient pressure for about 30 minutes. The pH of the soft biomass in the pretreatment tank is about 3.5. The pretreated biomass is then fed into a first hydrolysis tank, and bacteria in process water are added. The pH of the biomass in the first hydrolysis tank is about neutral and the retention time around 8 days. In the first hydrolysis tank biogas, such as methane and carbon dioxide is produced. The biogas may subsequently be further purified and utilized. The hydrolyzed biomass is drawn from the first hydrolysis tank and fed into a separator to obtain a liquid fraction and a solid fraction. The solid fraction is fed into a pressurized posttreatment tank where a posttreatment step is performed by heating the content to at around 170 degrees Celsius. After the posttreatment step the posttreated biomass may be separated by a separator, such as for example decantor and screw press, to obtain a liquid fraction and a solid fraction. At least a part of the liquid fraction or the entire liquid fraction may be recycled into the pretreatment tank or the first hydrolysis tank for reuse. The solid fraction is then subjected to a second hydrolysis step in a second hydrolysis tank by adding bacteria or enzymes or fungi. The retention time in the second hydrolyzation step is around 20 days. The biogas may be isolated and may be further purified and may be combined with the biogas produced in the first hydrolyzation step.

### FIGURE REFERENCES

SB. Soft biomass
NPS. Non-pressurized pretreatment step
PT. Pretreatment temperature
PB. Pretreated biomass
FHS. First hydrolyzation step
BH. Biomass hydrolysate
PPS. Pressurized posttreatment step
POT. Posttreatment temperature
POB. Posttreated biomass
SHS. Second hydrolyzation step
BG. Biogas
FSS. First separation step
SSS. Second separation step

## Claims

1. A method of producing biomass degradation products from soft biomass (SB), the method comprising the steps of
- providing a soft biomass (SB),
- pretreating the soft biomass (SB) in a pretreatment step (NPS) at a pressure below 2 bar by heating the soft biomass (SB) to at a pretreatment temperature (PT) between 65 to 100 degrees Celsius to obtain a pretreated biomass (PB),
- hydrolyzing the pretreated biomass (PB) in a first hydrolyzation step (FHS) to obtain a biomass hydrolysate (BH), and
- posttreating the biomass hydrolysate (BH) in a pressurized posttreatment step (PPS) by heating the biomass hydrolysate (BH) to a posttreatment temperature (POT) above 150 degrees Celsius to obtain a posttreated biomass (POB),
- hydrolyzing the posttreated biomass (POB) in a second hydrolyzation step (SHS),
wherein biogas (BG) is obtained from at least the first hydrolyzation step (FHS) or the second hydrolyzation step (SHS).

2. The method according to claim 1, wherein the pretreatment step (NPS) is non-pressurized.

3. The method according to claim 1 or 2, wherein the method further comprises separating the biomass hydrolysate (BH) in a first separation step (FSS) into a solid fraction and a liquid fraction.

4. The method according to any of the preceding claims, wherein the method further comprises separating the posttreated biomass (POB) in a second separation step (SSS) into a solid fraction and a liquid fraction.

5. The method according to any of the preceding claims, wherein the method further comprises recirculation of at least a part of a liquid fraction from any separation step (FSS, SSS) to any hydrolyzation step and/or the pretreatment step (NPS).

6. The method according to any of the preceding claims, wherein biogas is obtained from both the first hydrolyzation step (FHS) and from the second hydrolyzation step.

7. The method according to any of the preceding claims, wherein at least 65% hemicellulose is degraded.

8. The method according to any of the preceding claims, wherein at least 60% cellulose is degraded.

9. The method according to any of the preceding claims, wherein the amount of hemicellulose in the biomass hydrolysate (BH) is less than 40% by weight of the amount of hemicellulose in the soft biomass (SB).

10. The method according to any of the preceding claims, wherein the amount of cellulose in the biomass after the second hydrolyzation step (SHS) is less than 40% by weight of the amount of cellulose in the posttreated biomass (POB).

11. The method according to any of the preceding claims, wherein furfural and 5-HMF and 2-furioc acid are generated in a combined amount of less than 5% by weight.

12. The method according to any of the preceding claims, wherein xylose is obtained from the first hydrolyzation step (FHS).

13. The method according to any of the preceding claims, wherein lignin is obtained from the second hydrolyzation step (SHS) in a purity of more than 30%.

14. The method according to any of the preceding claims, wherein the pretreatment temperature (PT) is between 65 to 90 degrees Celsius, such as between 65 to 80 degrees Celsius or between 70 to 90 degrees Celsius.

15. The method according to any of the preceding claims, wherein the posttreatment temperature (POT) is between 150 to 230 degrees Celsius, such as between 170 to 210 degrees Celsius, such as between 180 to 200 degrees Celsius.

16. The method according to any of the preceding claims, wherein the pretreatment step (NPS) is performed for 2 hours or less.

17. The method according to any of the preceding claims, wherein the first hydrolyzation step (FHS) is performed for less than 20 days, such as less than 10 days.

18. The method according to any of the preceding claims, wherein the posttreatment step (PPS) is performed for less than 1 hour.

19. The method according to any of the preceding claims, wherein the second hydrolyzation step (SHS) is performed for less than 30 days.

20. The method according to any of the preceding claims, wherein the first hydrolyzation step (FHS) is performed by bacteria, enzymes and/or fungi.

21. The method according to any of the preceding claims, wherein the second hydrolysis step (SHS) is performed by bacteria, enzymes and/or fungi.

22. The method according to any of the preceding claims, wherein the enzymes are hydrolyzing enzymes, such as xylanases, cellulases, pectinases, lipases, arabinases or any combination thereof.

23. The method according to any of the preceding claims, wherein the bacteria are fermentative bacteria, such as ruminococcus albus, ruminococcus flavefaciens and/or fibrobacter succinogenes.

24. The method according to any of the preceding claims, wherein acid and/or food waste is added to the soft biomass (SB).

25. The method according to any of the preceding claims, wherein the acid is a weak acid.

26. The method according to any of the preceding claims, wherein the soft biomass (SB) is straw, corn stover, bagasse or any combination thereof.

27. The method according to any of the preceding claims, wherein the pH in the pretreatment step (NPS) is between 2 to 9.

28. The method according to any of the preceding claims, wherein the methane yield is more than 280 Nm3 per ton of soft biomass (SB) VS.
